Europäisches Patentamt

**European Patent Office**

**Office européen des brevets**

(11) Publication number : **0 590 752 A1**

(12) **EUROPEAN PATENT APPLICATION**

(21) Application number : **93301477.1**

(22) Date of filing : **26.02.93**

(51) Int. Cl.$^5$ : **A61M 15/02**

(30) Priority : **02.09.92 JP 234897/92**

(43) Date of publication of application :
**06.04.94 Bulletin 94/14**

(84) Designated Contracting States :
**DE FR GB IT**

(71) Applicant : **IZUMI LABORATORY CO., LTD.**
**1-25, Nanguu 2-chome**
**Okaya City, Nagano Prefecture (JP)**

(72) Inventor : **Miyasaka, Makiyoshi, Izumi**
**Laboratory Co., Ltd.**
**1-25, Nanguu 2-chome**
**Okaya City, Nagano Prefecture (JP)**

(74) Representative : **Leckey, David Herbert et al**
**Frank B. Dehn & Co., Imperial House, 15-19**
**Kingsway**
**London WC2B 6UZ (GB)**

(54) **Apparatus and method for producing liquid particles and negative ions.**

(57)  Ultra-fine liquid particles are produced which can reach pulmonary alveoli of a lung, to allow a medicament to be directly applied to pulmonary alveoli. Also negative ions are generated together with the ultra-fine liquid particles. Apparatus for producing ultra-fine liquid particles and negative ions comprises a fine liquid particle producing unit 2 having a dissolver 30 for mixing a medicament with a liquid to prepare a solution and generating fine liquid particles from this solution ; a fan 3 for introducing air at a wind velocity of 0.5 - 50 m/sec into the unit 2 simultaneously with the generation of the fine liquid particles to produce fine liquid particle mixed air ; and a separator 4 for removing fine liquid particles with the diameter of more than 0.3 $\mu$m to provide ultra-fine liquid particle mixed air containing ultra-fine liquid particles with the diameter of 0.3 $\mu$m or less. Alternatively particles of more than 1 $\mu$m may be removed, if production of negative ions only is sought.

Fig.1

This invention relates to a method and apparatus for producing fine liquid particles and negative ions in the air, and a method and apparatus for utilizing the produced fine liquid particles and negative ions.

Conventionally, medical nebulizers being clinically used may be classified into an ultrasonic-type, a compressor-type, an intermittent pressure breathing type and so on. The nebulizer may also be called a spray. The inhalation of a medicament by using a nebulizer device is referred to as an aerosol inhalation method, while the inhalation of aerosoled water only for purpose of humidifying an air path is referred to as a humidification medical treatment. These methods are provided for spraying a medicament or moisture as fine particles and sending these particles deep into an air path and lungs. Recently, an ultrasonic nebulizer capable of uniformly generating ideal fine particles of several microns is often used because of less physical burden on a patient.

This ultrasonic nebulizer in general produces liquid particles of approximately 0.4 $\mu$m - 15 $\mu$m in diameter. An average diameter is 2.7 $\mu$m. Conventionally, this ultrasonic nebulizer has been used to have a patient inhale a medicament from the pharynx to the larynx, trachea and bronchia or humidify these parts.

In relation to this ultrasonic nebulizer, when water particles are diffused in the air, that is, when water particles are broken up in the air, the water particles are charged positive while ambient air is charged negative, thus resulting in an ionization phenomenon of air. This air ionization phenomenon removes positive water particles in the air for some reason, and accordingly floating negative ions prevail in the air. This is generally known as a Lenard effect. and may occur in particular areas such as around a water fall and a rapid stream, like the upstream of a river and an area where it is currently raining. These negative ions are believed to have effects of stabilizing the mind and enhancing the respiratory organs. Previously, there has been no other method than relying upon negative ions naturally existing in the air, however, the long-term investigations and advancement of electronic engineering have developed a negative ion (negative potential) curing means as an apparatus which is capable of arbitrarily supplying negative ions irrespective of the situation of ions in the air.

However, the size of water particles generated by an ultrasonic nebulizer conventionally used in clinical purposes ranges from 0.4 $\mu$m to 15 $\mu$m, the average of which is 2.7 $\mu$m, and it is said that water particles of this size will reach bronchiole but not pulmonary alveoli. For this reason, a conventional ultrasonic nebulizer cannot be used for the direct supply of water particles into pulmonary alveoli in expectation of achieving humidification effects to facilitate a patient to spit out phlegm. It is also impossible to apply a water soluble medicament such as an antasthmatic and antibiotics, by way of example, in order for a patient to absorb it through pulmonary alveoli.

Further, according to the foregoing negative ion treating device, a large amount of negative ions are generated so that these negative ions can be easily supplied to cells and a negative ion effect is produced in an organism, resulting in exerting the influences in terms of body fluid, cell and nerve on the whole body, further leading to treating a variety of diseases and improving the health. However, a large amount of ozone ($O^3$) is produced in return for the generation of negative ions. It is believed that even a very small amount of ozone is hazardous to the human body.

The present invention has been made in view of the problems mentioned above, and its first object is to clarify the diameter of liquid particles which can reach pulmonary alveoli of the lung and to provide a method and apparatus for generating liquid particles which can reach the pulmonary alveoli and a method and apparatus for utilizing the thus produced liquid particles.

A second object of the present invention is to provide a method and apparatus for generating negatively charged ions contingent to shaping of liquid particles for providing the same with the diameter below a predetermined value, and a method and apparatus for utilizing the generated negative ions.

To achieve the above object, the present invention in one preferred embodiment generates liquid particles and negative ions by the steps of generating fine liquid particles from a liquid by a fine liquid particle producing unit; mixing said fine liquid particles with air at an air velocity of 0.5-50 m/sec to produce fine liquid particle mixed air simultaneously with said fine liquid particle generating step; passing said fine liquid particle mixed air through a separator to substantially remove fine particles, the diameter of which is larger than 0.3 $\mu$m to produce ultra-fine liquid particle mixed air; and generating, per 1M$^3$ of ultra-fine liquid particle mixed air, $5.0 \times 10^8$ or more of ultra-fine liquid particles with the diameter of 0.3 $\mu$m or less which can reach pulmonary alveoli of the lung as well as generating negative ions.

Where it is desired principally to provide negative ions, particles of diameters over 1.0 $\mu$m may instead be removed.

Some preferred embodiments of the present invention will now be described by way of example with reference to the accompanying drawings, in which:

Fig. 1 illustrates the structure of an apparatus which embodies a method of generating ultra-fine liquid particles and negative ions which can reach pulmonary alveoli, according to the present invention;

Fig. 2 illustrates the structure of an apparatus for producing fine liquid particles according to another embodiment of the present invention;

Fig. 3 illustrates the structure of an apparatus for producing fine liquid particles according to a further embodiment of the present invention;

Fig. 4 illustrates the structure of an apparatus for producing fine liquid particles according to a yet further embodiment of the present invention;

Fig. 5 illustrates the structure of an apparatus for producing liquid water particles according to a yet further embodiment of the present invention;

Fig. 6 is a cross-sectional view showing part of an air channel shown in Fig. 5;

Fig. 7 is a cross-sectional view showing a dispersing condition of a liquid spurting from a nozzle of the air channel;

Fig. 8 illustrates the structure of an apparatus which embodies a method of utilizing liquid particles and negative ions, according to the present invention;

Fig. 9 illustrates the structure of an apparatus for producing liquid particles and negative ions according to another embodiment of the present invention;

Fig. 10 illustrates the structure of an apparatus for producing liquid particles and negative ions according to another embodiment of the present invention; and

Fig. 11 illustrates the structure of an apparatus which embodies the method of utilizing liquid particles and negative ions, according to the present invention.

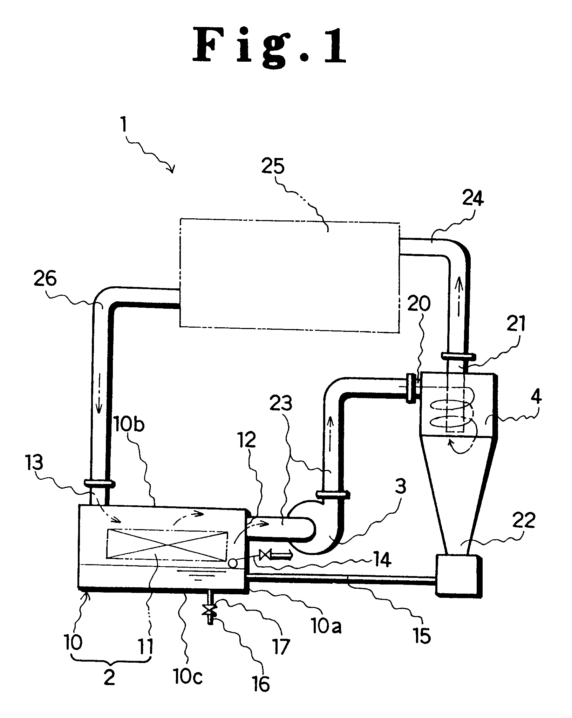

Referring first to Fig. 1, an apparatus for producing liquid particles and negative ions comprises a fine water particle producing unit 2 for generating fine liquid particles from a liquid; a fan 3 for introducing air into the apparatus 1 at a velocity of 0.5 - 50 m/sec simultaneously with the generation of fine liquid particles by the fine liquid particle producing unit 2; and a separator 4 for removing fine liquid particles having a diameter larger than 0.3 μm from the air mixed with the fine liquid particles to provide air mixed with ultra-fine water particles. With this structure, the apparatus 1 produces ultra-file liquid particle mixed air containing more than $5.0 \times 10^8$ per 1m³ of the ultra-fine liquid particles with a diameter of 0.3 μm or less which can reach pulmonary alveoli of the lung, as well as negative ions.

The fine liquid particle producing unit 2 includes a tank 10 in which an ultrasonic humidifier 11 is arranged. This tank can maintain a substantially air tight condition and is typically made of a material which is resistant to corrosion due to a medicament or solution to be contained therein, e.g., stainless steel or plastic, although not particularly specified. The shape of the tank 10 is not limited in particular and may be cylindrical, square, rectangular, spherical and so on. A lateral face 10a and the top face 10b of the tank 10 are formed with an air outlet port 12 and an air inlet port 13, respectively, which are connectable with external apparatus through pipes and ducts.

The lateral face 10a of the tank 10 is also provided with a ball tap valve 14 for receiving a liquid and a return pipe 15. Further, on the bottom face 10c of the tank 10, there is attached a drain pipe 16 having a valve 17.

As stated above, the ultrasonic humidifier 11 is arranged in this tank 10. This ultrasonic humidifier 11 is designed to generate liquid particles at relatively low temperatures by introducing a liquid (mainly water is used) into the tank 10, vibrating the liquid, and generating heat by friction. Since many ultrasonic humidifiers 11 of this type are presently available on the market, an appropriate one adapted to the power supply of a particular area or country may be selectively utilized.

Although in this embodiment, an ultrasonic humidifier is employed, the present invention is not necessarily limited to this type of humidifier. Alternatively, a humidifier utilizing so-called Joule heat may be employed. In this case, since the temperature of generated liquid particles is relatively high, the particles are cooled down by contacting with air in winter, while temperature adjustment may often be required for the particles in summer. Anyway, the employment of such thermal means for generating liquid particles results in providing a compact humidifier as compared with means for mechanically generating liquid particles.

The fan 3 used herein is not particularly limited as long as it provides sufficient pressure and flow to ensure an air velocity ranging from 0.5 m/sec to 50 m/sec Preferably, the fan 3 is made of a material resistant to corrosion caused by a used liquid, generally, of stainless steel or synthetic resin.

The separator 4 is provided for removing fine liquid particles having a diameter of more than a predetermined value from the fine liquid particle mixed air to select ultra-fine liquid particles which can reach pulmonary alveoli. The separator 4 may be of any type as long as it has this function. In this embodiment, as the separator 4, a cyclone is employed which is selected as having an appropriate diameter and length in accordance with the diameter of target ultra-fine liquid particles. More specifically, for the separator 4 of this embodiment, a cyclone is used which removes fine liquid particles, the diameter of which is larger than 0.3 μm. The material used for the separator should be resistant to oxidation. The cyclone 4 serving as a separator is provided with a supply port 20 in a lateral face thereof, an emitting port 21 in the top face, and a drain port 22 in the bottom face.

In the separator of this embodiment, when fine liquid particles having the diameter of more than 0.3 μm are to be removed, a large amount of negative ions are generated. Although the principle of generating such negative ions is not known, the following assumption can be made. At the same time fine liquid particles are generated from a liquid by the fine liquid particle producing unit 2, air is fed to these fine liquid particles at a velocity of 0.5 - 50 m/sec to produce fine liquid particle mixed air, wherein part of the fine liquid particles become positive ions due to the Lenard effect while part of air surrounding these ionized liquid particles become negative ions. In this event, an electrically balanced condition is established. The fine liquid particle mixed air including negative charged air ions and positive charged liquid particles is sent to the separator 4 which removes positively charged fine liquid particles having a diameter of more than 0.3 μm, resulting in losing the balance of air ions, and providing ultra-fine liquid particle mixed air including a large amount of negative ions.

The foregoing fine liquid particle producing unit 2, the fan 3 and the separator 4 are connected in the following manner. The air outlet port 12 of the tank 10 in the fine liquid particle producing unit 2 is connected to the supply port 20 of the separator 4 through a duct 23 and the fan 3. The drain port 22 of the separator 4 is connected to the return pipe 15 to provide a return path to the tank 10. The emitting port 21 formed in the top face of the separator 4 is connected to the air inlet port 13 of the tank 10 in the fine liquid particle producing unit 2 through a duct 24, an individual treatment room 25 and a duct 26.

If there is no treatment room available and a patient is directly supplied with ultra-fine particle mixed air through an air supply mask or the like, the separator 4 may be provided with an air supply mask attached on the emitting port 21 thereof through a tube. Additionally, an air filter or the like for preventing dust from entering may be attached on the air inlet port 13 of the tank 10.

Next, a method of producing liquid particles and negative ions according to the present invention will be described in connection with the foregoing apparatus 1.

First, after confirming required conditions being satisfied, e.g., whether or not a liquid exists up to a predetermined level in the tank 10 of the fine liquid particle producing unit 2, the fan 3 is rotated while the ultrasonic humidifier 11 is operated. The ultrasonic humidifier 11 introduces the liquid into the tank 10 to generate fine liquid particles which are dispersed in air supplied by the fan 3, thereby forming fine liquid particle mixed air comprising positively ionized fine liquid particles due to the Lenard effect and negatively ionized air therearound which in turn is supplied through the duct 23 and the fan 3 from the supply port 20 to the separator 4 in the tangential direction. The fine liquid particle mixed air in the tank exhibits a revolving flow, and positively ionized fine liquid particles having the diameter larger than a predetermined value, for example, 0.3 μm, strike against the inner peripheral wall of the separator 4, fall along the wall, and return to the tank 10 from the drain port 22 through the return pipe 15. Mixed air including ultra-fine liquid particles having the diameter of 0.3 μm or less and negative ions is supplied to the individual treatment room 25 from the emitting port 21 through the duct 24. This mixed air contains more than $5.0 \times 10^8$ per 1 $m^3$ of ultra-fine liquid particles having the diameter of 0.3 μm or less and $1.25 \times 10^9$ of negative ions. A patient, when breathing this mixed air in the individual treatment room 25, the ultra-fine liquid particles can be sent to pulmonary alveoli of the lung, which is naturally accompanied by the supply of negative ions to the patient. The ultra-fine liquid particle mixed air, after the service, is again supplied from the air inlet port 13 to the tank 10 in the fine liquid particle producing unit 2 through the duct 26, thereby performing a circulating operation.

Next, experiments are made for confirming whether or not the ultra-fine liquid particles produced by the method of producing liquid particles and negative ions according to the present invention actually reach pulmonary alveoli of lung. The experiments are performed in the following manner.

1. Apparatus for producing liquid particles and negative ions in accordance with the present invention was supplied with water, and the size or diameter of generated ultra-fine particles was measured with a fine particle counter KB-01B (manufactured by Rion Co., in Tokyo).

2. A solution of tritium thymidine (3H-6-thymidine NEN, USE, specific activity 557 GBq/mM, 37MBq), which is a radio isotope (RI) dissolved by 10 ml of pure water was supplied to the tank 10 of the fine liquid particle producing unit 2 to generate tritium thymidine dissolved water particles. Then, five ddY-line mice (weight: 15g, male and female) were placed in an air tight vinyl bag corresponding to the individual treatment room 25 to have them breathe the tritium thymidine dissolved water particles for 45 minutes. For comparison purposes, five identical mice were left in the laboratory. Immediately, these ten mice were killed and the lower lobe tissues of the right lung were collected and rapidly frozen by a cryopexy apparatus (JFD-RFA manufactured by Japan Electronics, Japan) using liquid nitrogen at a temperature of -196°C. After the frozen tissues were kept for 72 hours under -80°C with an acetone by Frost Substitution Unit (Reichect-Jung CS-Auto), those were returned to the normal temperature and embedded in Luveak 812 (Ouken). The tissues were fixed for one (1) hour each with 0.1M phosphate buffer (pH 7.4), 2.5-% glutaraldehyde and 1% osmium tetroxide, then dehydrated by series alcohol and embedded in Luveak 812.

Sections 2μm thick were made by Porter-Blum MT-2B type Ultra-Microtome (Manufactured by DuPont-Sorvall, USA) and put on the slideglass. Chemically fixed materials were used with water but frozen materials applied ethylene glycol instead of water.

Konica NR-M2 emulsion is used for Radioautography. The frozen materials were treated with wire-loop method of dry mounting emulsion and chemically fixed ones were treated with dip method of wet mounting emulsion. They had exposed for 70 days at 4°C in the refrigerator, then developed by SDX-1 developer and were established.

They were stained with 0.1 % of toluidine blue, and observed with OLYMPUS Vanox AHB-LB Optical Microscope by applying falling light and transmission light then took photographs.

The characteristics of ultrafine water particles have no sticky feeling and wetness, for example, no moisture collected to the glasses. Water particles measured by Particle Counter and the results are shown as follows. 93.3 % of particles are less than 0.3 μm in diameter, 4.2 % is 0.3 to 0.5 μm, 1.5 % is 0.5 to 1.0 μm, and 1 % only for more than 2 μm. By an observation of lightmicroscopic radioautography, chemically fixed samples and emulsion wet-mounting samples were showing rare silver grains but observed a few silver grains on epithelial cells of the bronchioles and on a nucleus of alveolar epithelium cells. On the other hand, the soluble compound radioautograph of frozen samples and dry-mounting samples were observed a lot of silver grains on the bronchioles, nucleus of the alveolar epithelia and the connective tissues. This results show that $^3$H-thymidine is absorbed into alveolar epithelia cells through the bronchioles. And as the results, it was proved that $^3$H-thymidine, i.e., RI in the ultrafine particles reached widely to the alveoli, and hence it indicates that ultrafine particles produced by the liquid particle and negative ions producing apparatus 1 of the present invention can effectively reach the alveoli. Silver grains on the soluble compound radioautograph of chemically fixed samples and wet-mounting samples are dotted in DNA-synthesis S-term cells and those are no so many, but a lot of silver grains on the soluble compound radioautography of frozen samples and dry-mounting samples will show soluble compound.

According to the above results, it was proved that the ultrafine water particles can reach the mouse alveoli directly.

Since the size of the pulmonary alveolus of a mouse is similar to that of a human, it can be thought from this result of the experiment that the apparatus 1 for producing liquid particles and negative ions is expected to provide humidification effects also for the respiratory organs of humans, and removal of sputum will be easy. Furthermore, ultrafine water soluble substances including such as asthma treatment drugs, antibiotics, and other medicaments can be expected to be administered through the lung.

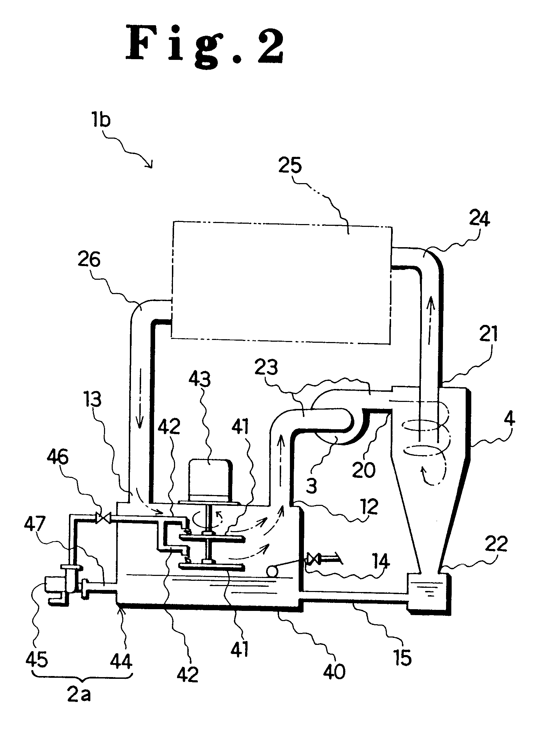

Incidentally, although in the apparatus 1 for producing liquid particles and negative ions of this embodiment, the fine liquid particle producing unit 2 employing the ultrasonic humidifier 11 has been explained, the present invention is not limited to this. Alternatively, the present invention may employ an apparatus lb for producing a liquid particle and negative ion comprising a fine liquid particle producing unit 2a as shown in Fig. 2. This apparatus comprises a fine liquid particle generator 44 including a tank 40; at least one disk 41 horizontally rotatably arranged in the tank 40; a liquid supply pipe 42 positioned above the disk 41 and mounted on the tank 40; and a driving unit 43 for rotating the disk 41, and a liquid supply unit 45 for supplying the liquid supply pipe 42 with a liquid at a gage pressure of -0.20-3.5 Kg/cm². In Fig. 2, reference numeral 46 designates a flow control valve, and 47 a pull-out pipe. The rest of the structure in Fig. 2 similar to the constituents of Fig. 1 will be designated the same reference numerals, and explanation thereof will be omitted.

Now, referring again to Fig. 2, the fine liquid particle producing unit 2a rotates the disk 41 while the liquid supply unit 45 supplies the same with a liquid, thereby generating fine liquid particles.

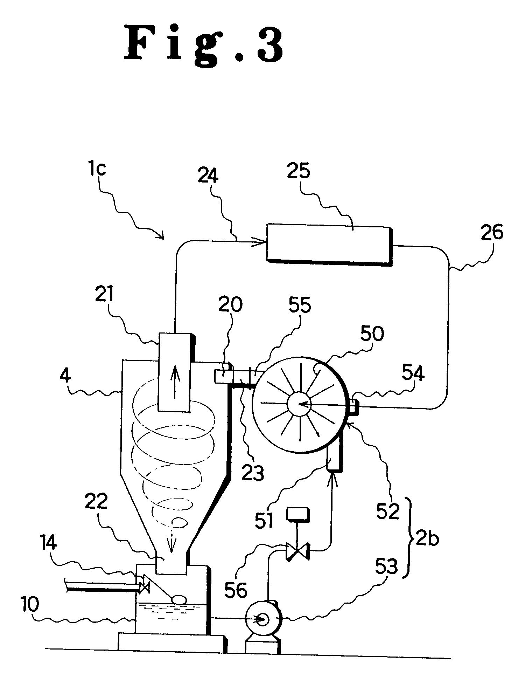

Alternatively, as shown in Fig. 3, an apparatus lc may be provided with a fine liquid particle producing unit 2b which comprises an impeller 50 adapted to rotate at high speeds to transport air at from 0.5 to 50m/s; a fine liquid particle generating unit 52 provided with a liquid supply tube 51; and a liquid supply unit 53 for supplying the impeller 50 of the fine liquid particle generating unit 52 with a liquid at a gage pressure of -0.20 - 3.5 Kg/cm² through the liquid supply tube 51. In Fig. 3, reference numeral 54 designates an air inlet port; 55 an air outlet port; and 56 a flow control valve. The remaining constituents in Fig. 3 common to those of Fig. 1 will be designated the same reference numerals, and explanation thereof will be omitted.

This fine liquid particle producing unit 2b rotates the impeller 50 of the fine liquid particle generating unit 52, which serves as a turbine fan, and supplies the impeller 50 with a liquid by the liquid supply unit 53, thereby transporting air while generating fine liquid particles.

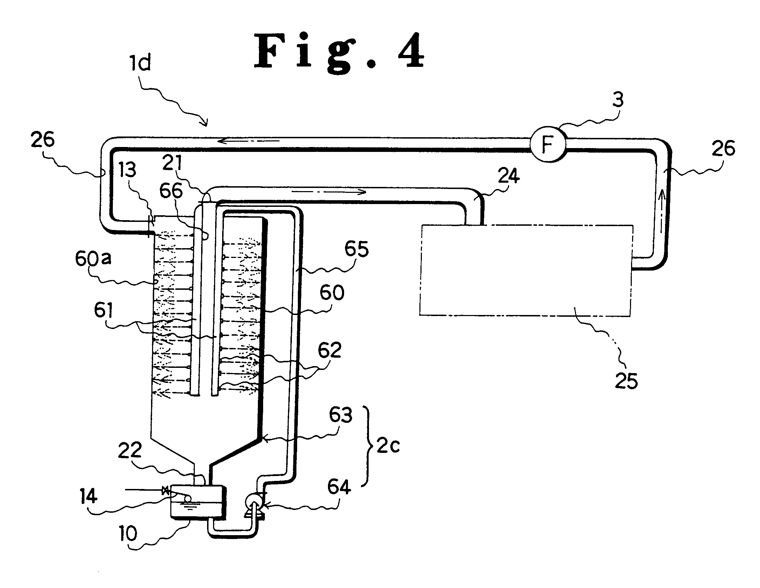

Further, as shown in Fig. 4, a fine liquid particle producing unit 2c of an apparatus 1d may comprise a liquid spouting unit 63 and a liquid supply unit 64. Specifically, the liquid spouting unit 63 strikes a liquid onto a lateral wall 60a of a liquid spouting unit body 60 located 10-150 cm away from a multiplicity of nozzles having the diameter ranging from 0.2 to 8 mm arranged around a spouting pipe 61 which is arranged in a central portion of the liquid spouting unit body 60, thereby generating an extremely large amount of fine liquid particles. The body 60 has a cyclonic function so as to remove liquid particles having a diameter over a predetermined value. The liquid supply unit 64 supplies the liquid from the nozzles 62 at a gage pressure of 0.5-3.5 Kg/cm². In Fig. 4, reference numeral 65 designates a liquid supply pipe, and 66 an emitting pipe. The remaining constituents in Fig. 4 common to those in Fig. 1 will be designated the same reference numerals, and explanation thereof will be omitted.

This fine liquid particle producing unit 2c operates the liquid supply unit 64 to strike the liquid from the nozzles 62 onto the lateral wall 60a of the liquid spouting unit body 60 to generate fine liquid particles.

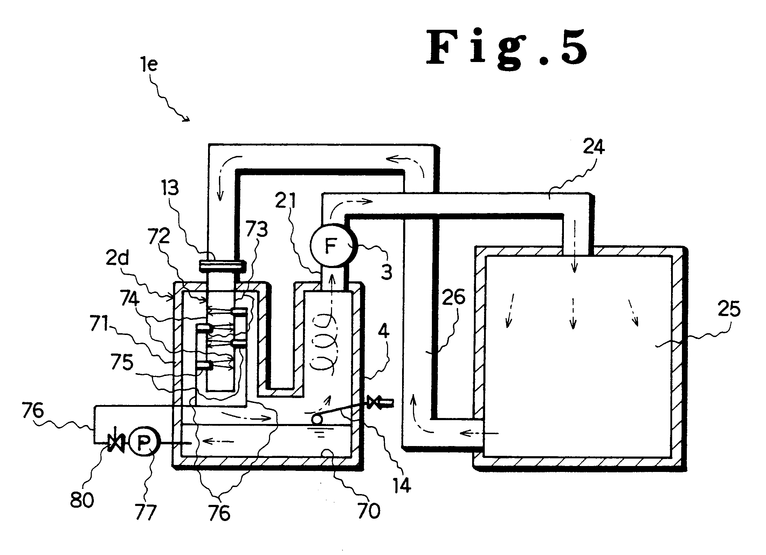

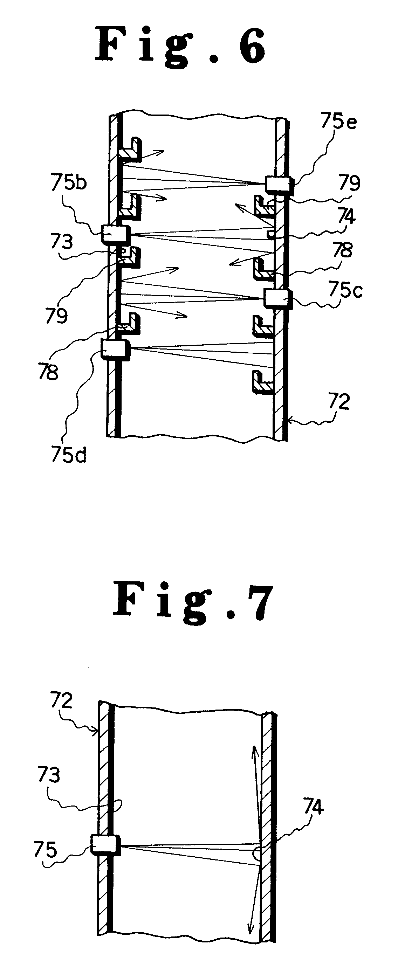

Referring next to Fig. 5, another apparatus le according to the present invention is equipped with a fine liquid particle producing unit 2d which comprises a tank 70; an air channel 72 accommodated in a cylinder 71 arranged on this tank 70; nozzles 75 formed in a wall surface 73 of the air channel 72 for spouting a liquid onto the opposite wall face 74; a supply pipe 76 for supplying the respective nozzles 75 with a liquid; and a pump 77 for feeding this supply pipe 76 with the liquid. A separator 4 is further arranged above the tank 70 and communicates with the air channel 72 in a manner that substantial air tight condition can be maintained. The foregoing nozzles 75 are sequentially aligned in the order of 75e, 75d, 75c and 75b such that the axial direction of the nozzles 75 is perpendicular to the wall surface 73 of the air channel 72. On the other hand, the opposite wall surface 74 of the air channel 72 is provided with an emitted liquid receiving plate 78 which receives a liquid which was spouted from the nozzles and is not dispersed but falls down on the opposite wall surface 74. Liquid spray spouted from, for example, the nozzle 75e falls down on the opposite wall surface 74 to form a liquid film over the wall surface 74, and other liquid spray strikes from another nozzle 75b on this liquid film, where the liquid film serves as a cushion to alleviate collision energy, resulting in preventing the generation of fine liquid particles from decreasing. Further, a control plate 79 is arranged on the opposite wall surface 74 above the emitted liquid receiving plate 78. This control plate 79 is provided for controlling the dispersing direction of the liquid after having stricken on the opposite wall surface 74 such that fine liquid particles spouted from other nozzles 75 are protected from being absorbed by such a liquid film formed on the surface. The emitted liquid receiving plate 73 also acts to control a dispersing condition of the liquid spray in a manner similar to the control plate 79, as shown in Fig. 7. In Fig. 5, reference numeral 80 designates a flow control valve. The remaining constituents in Fig. 5 common to those in Fig. 1 will be designated the same reference numerals, and explanation thereof will be omitted.

Next, the measurement results will be shown with respect to negative ions produced by the method of producing liquid particles and negative ions according to the present invention.

Embodiment 1

A sensor of an air ion counter 83-1001A (manufactured by Dan Science) is set up at a location spaced by 0.6 m from the emitting port 21 of the separator 4 in the apparatus 1 for producing liquid particles and negative ions shown in Fig. 1. Then, the apparatus 1 is driven to produce ultra-fine water particle mixed air, and the respective numbers of positive ions and negative ions contained in this ultra-fine water particle mixed air are continuously counted for 20 minutes. Incidentally, a cyclone for separating liquid particles, the diameter of which exceeds 1 $\mu$m is selected for the separator 4.

Embodiment 2

Using the apparatus Ib shown in Fig. 2, the numbers of positive ions and negative ions are counted in a manner similar to Embodiment 1 at a location spaced by 0.6 m from the emitting port 21 of the separator 4 in the apparatus 1b.

Embodiment 3

Using the apparatus Ic shown in Fig. 3, the numbers of positive ions and negative ions are counted in a manner similar to Embodiment 1 at a location spaced by 0.6 m from the emitting port 21 of the separator of the apparatus 1c.

Embodiment 4

Using the apparatus 1d shown in Fig. 4, the numbers of positive ions and negative ions are counted in a manner similar to Embodiment 1 at a location spaced by 0.6 m from the emitting port 21 of the liquid spouting unit 63 in the apparatus 1d.

Embodiment 5

Using the apparatus le shown in Fig. 5, the numbers of positive ions and negative ions are counted in a manner similar to Embodiment 1 at a location spaced by 0.6 m from the emitting port 21 of the separator 4 in the apparats 1e.

Next, the following measurements are taken for comparing the results with the foregoing Embodiments 1 - 5.

Comparative Example 1

A nebulizer of a type NE-V110B (manufactured by Tateishi Omron, Japan) available on the market is used to generate negative ions and positive ions. The numbers of generated positive and negative ions are counted at a location spaced by 0.01 m from a negative ion emitting port of the nebulizer.

Comparative Example 2

The numbers of negative ions and positive ions are counted at a location spaced by 0.6 m from a blowout port of an air conditioner normally available on the market.

Comparative Example 3

In the laboratory where the measurements of the foregoing embodiments and comparative examples were taken, a sensor of an air ion counter is set up in a manner similar to Embodiment 1 to count the numbers of positive ions and negative ions in the air in the laboratory.

Comparative Example 4

The sensor of the air ion counter is set up at an outdoor location near the laboratory to count the numbers of positive and negative ions in the external air.

The following Table 1 indicates the measurement results.

Table 1

| | Negative Ions (per $m^3$) | Positive Ions (per $m^3$) |
|---|---|---|
| Embodiment 1 | $7.0 \times 10^{10}$ | $2.0 \times 10^{9}$ |
| Embodiment 2 | $3.5 \times 10^{10}$ | $1.5 \times 10^{8}$ |
| Embodiment 3 | $5.0 \times 10^{10}$ | $1.0 \times 10^{6}$ |
| Embodiment 4 | $10.0 \times 10^{10}$ | $15.0 \times 10^{8}$ |
| Embodiment 5 | $11.5 \times 10^{10}$ | $13.5 \times 10^{8}$ |
| Comparative Example 1 | $0.4 \times 10^{8}$ | $2.0 \times 10^{8}$ |
| Comparative Example 2 | $2.5 \times 10^{8}$ | $0.4 \times 10^{8}$ |
| Comparative Example 3 | $2.2 \times 10^{8}$ | $1.8 \times 10^{8}$ |
| Comparative Example 4 | $1.8 \times 10^{8}$ | $1.6 \times 10^{8}$ |

Note: Room Temperature and External Air Temperature: 25 - 30°C

Humidity: 50 - 80 %

As is apparent from Table 1, the method of the present invention can generate an amount of negative ions 10 - 25 times as much as the nebulizer used for Comparative Example 1 whereas it generates an amount of positive ions which is merely a bit more than the positive ions generated by the nebulizer or those naturally existing in the laboratory and in the external air.

Next, for measuring the concentration of ozone, a sensor of an ozone measuring instrument of a type EG-2001G (manufactured by Ebara Corporation, Japan) is placed respectively at a location near the emitting port 21 of the apparatus of the present invention employed for the foregoing Embodiments 1 - 5; at a location near a discharge port of the nebulizer employed for Comparative Example 1; at a location near the blow-out port of the air-conditioner employed for Comparative Example 2; in the laboratory; and outdoors as in Comparative Examples 3 and 4. The following Table 2 indicates the measurement results.

Table 2

| | Ozone $(O^3)$ $10^{-6}$ % |
|---|---|
| Embodiment 1 | Not Available |
| Embodiment 2 | Not Available |
| Embodiment 3 | Not Available |
| Embodiment 4 | Not Available |
| Embodiment 5 | Not Available |
| Comparative Example 1 | 1,250 |
| Comparative Example 2 | Not Available |
| Comparative Example 3 | Not Available |
| Comparative Example 4 | Not Available |

Note: Room Temperature and External Air Temperature: 25 - 30°C

Humidity: 50 - 80 %

It is assumed that the ultra-fine liquid particles generated by the present invention contain a certain amount of ozone which is equal to the amount of ozone believed to be generally contained in the natural air, i.e., $10^{-6}$ %, however, the amount of ozone could not be measured, due to the accuracy of the instrument, except for ozone generated by the nebulizer (Comparative Example 1).

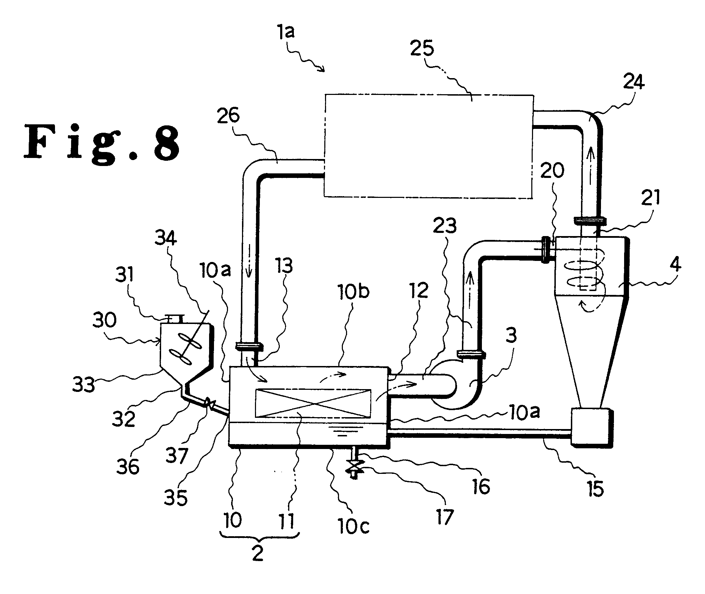

For directly supplying pulmonary alveoli with a medicament by means of the thus structured apparatus for producing liquid particles and negative ions 1, 1b, 1c, 1d or 1e, an apparatus 1a for utilizing liquid particles and negative ions as shown in Fig. 8 may be used.

Specifically, this utilizing apparatus 1a is provided with a dissolver 30 in place of the ball tap valve 14 of the apparatus for producing liquid particles and negative ions. This dissolver 30 comprises a tank having a medicament supply port 31 and a pull-out port 32; an agitator 34 mounted in the dissolver 30; a supply path 36 connecting the pull-out port 32 and a receiving port 35 of a tank 10; and a valve 37 arranged in a midway location of the supply path 36. As a medicament and a liquid are supplied into the tank 33 through the medicament supply port 31, the agitator 34 is operated to mix the medicament with the liquid, and this solution is supplied to the tank 10. The dissolver 30 is made of a material which is resistant to corrosion caused by the used medicament and liquid. The medicament may be solid, liquid or gas. If a medicament is liquid and hence need not be dissolved by a liquid in particular, the dissolver 30 is not necessary. It may be supplied as it is to the tank 10 of the fine liquid particle producing unit 2.

Explaining the operation of the apparatus 1a for utilizing liquid particles and negative ions, the valve 37 in the supply path 36 is first closed, and a medicament necessary for a particular treatment and a liquid as a solvent of this medicament are supplied to the tank 33 of the dissolver 30 through the medicament supply port 31. Next, the agitator 34 is operated to mix the medicament with the liquid to prepare a solution. When this solving operation is completed, the agitator 34 is stopped, the valve 37 is opened, and the solution is supplied from the pull-out port 32 to the tank 10 of the fine liquid particle producing unit 2 through the supply path 36 and the receiving port 35. Further, after producing ultra-fine liquid particle mixed air is produced, if a patient is instructed to directly breathe this mixed air through an air supply mask or the like, ultra-fine water particles containing the medicament having the diameter not more than 0.3 μm can be fed to pulmonary alveoli.

If it is desired to principally provide the negative ions rather than the ultra-fine liquid particles of 0.3 μm or less in diameter which can reach pulmonary alveoli, a cyclone for removing fine particles of more than 1.0 μm in diameter is sufficient for this purpose and therefore is selected for the separator 4. Then, by supplying water to the foregoing apparatus 1, 1b, 1c, 1d or 1e for producing liquid particles and negative ions, the apparatus can produce ultra-fine liquid particle mixed air containing a large quantity of negative ions and particles of less than 1 µm in diameter which are applied to patients in respective individual treatment rooms 25. It is well known that the negative ions present the effects as shown in the following Table 3.

Table 3

| | Action of Air Ions | |
|---|---|---|
| Item | Effect of Negative Ions | Effect of Positive Ions |
| Blood Vessel | Expanded | Contracted |
| Blood Pressure | Normalized | Higher |
| Blood (PH) | Alkalized | Acidified |
| Bone | Strong | Fragile |
| Urine | Urination Promoted Urinary Nitrogen Increased | Urination Restricted Urinary Nitrogen Decreased |
| Respiration | Calm and Relaxed | Promoted and Difficult |
| Pulse | Decreased | Increased |
| Heart | Easy to Work | Hard to Work |
| Fatigue | Recovery Promoted | Recovery Delayed |
| Autonomic Nerve | Relieving Functions | Tensing Functions |
| Growth | Favorably Promoted | Bad and Delayed |

In addition, when the ultra-fine liquid particle mixed air is supplied together with ordinary air, minute dust, mould, bacteria and so on attach to the liquid particles, with the result that purification and bacteria elimination are realized. Further, under a circumstance where such ultra-fine liquid particles are floating in the air, while the liquid particles purify air, no object in the vicinity is wetted by the liquid particles in spite of the fact that they contain moisture.

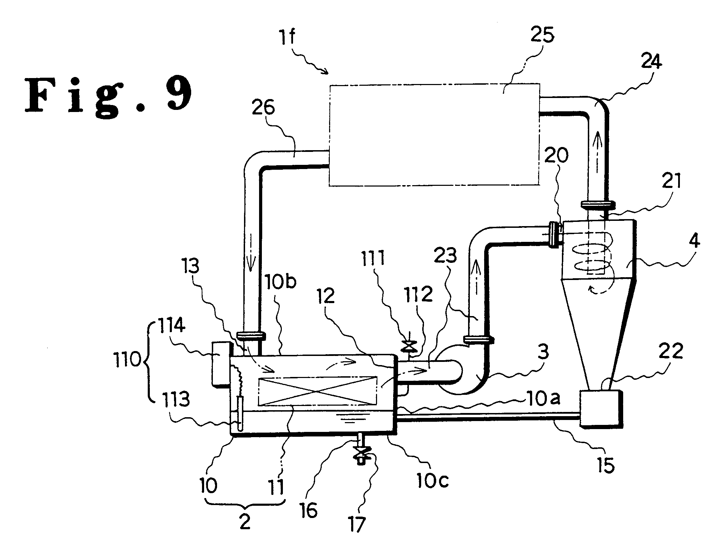

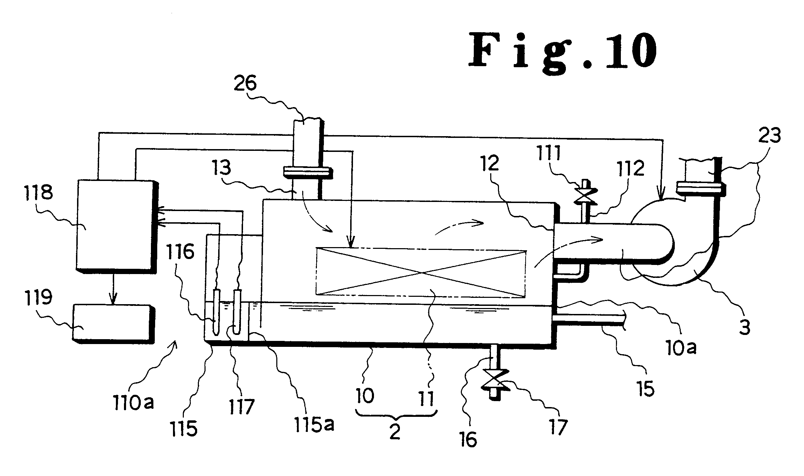

Figs. 9 and 10 illustrate the structure of an apparatus for producing liquid particles and negative ions, generally designated by 1f, which does not generate negative ions with liquids other than water. This apparatus is provided with a water detecting sensor 110 and a supply path 112 equipped with a valve 111 in a tank 10, in addition to the structure of the apparatus 1 shown in Fig. 1.

Referring to Fig. 9, the water detecting sensor 110 may be a conductivity meter which comprises a housing 113 containing a sensor therein which is arranged in water in the tank 10; and a control indicator 114 connected to this housing 113 for amplifying a signal indicative of a conductivity and indicating this conductivity as well as controlling the fine liquid particle producing unit 2 and the fan 3 in accordance with the conductivity. The conductivity of water ranges from 150 to 250 micro Siemens (µs/cm) for ordinary water, and from 20 to 30 micro Siemens (µs/cm) for distilled water. Therefore, a liquid, the conductivity of which lies between 10 and 500 micro Siemens (µs/cm) may be determined to be water, taking into account a slight tolerable error margin. In this manner, if the conductivity of a liquid in the tank 10 is in the range between 10 and 500 micro Siemens (µs/cm), the liquid in the tank 10 is determined to be water, and the apparatus 1f normally operates to utilize negative ions. If a liquid, the conductivity of which is out of the above range, is not determined to be water, a stop signal is generated from the control indicator 114 to at least one of the fine liquid particle producing unit 2 and the fan 3, thereby stopping the operation to prevent negative ions from being generated, thus avoiding damages due to a liquid other than water.

Referring to Fig. 10, the water detecting sensor 110a is formed of a combination of a specific gravity meter and a viscosimeter. This water detecting sensor 110a has the specific gravity meter 116 and the viscosimeter 117 accommodated in a meter box 115 arranged on a lateral surface of the tank 10. The meter box 115 communicates with the tank 10 through a gate 115a interposed therebetween. The water detecting sensor 110a receives signals from the specific gravity meter 116 and the viscosimeter 117 indicative of measured values at a controller 118, and displays these measurement signals on a display unit 119 as well as generates an operation signal or a stop signal from the controller 118 to the fine liquid particle producing unit 2 and the fan 3. It is known that water at normal temperatures exhibits its specific gravity and viscosity within respective predetermined ranges, so that if measured specific gravity and viscosity values of a liquid are out of respective predetermined ranges, the liquid is determined to be a liquid other than water, and the liquid particle producing unit 2 and the fan 3 are supplied with a stop signal to prevent them from operating. On the other hand, if measured values of these properties are within the respective predetermined ranges, the liquid is determined to be water so that the liquid particle producing unit 2 and the fan 3 are supplied with an operation signal to allow them to operate.

Incidentally, this embodiment has been explained for a case where the water detecting sensor employs a conductivity meter and a combination of a specific gravity meter and a viscosimeter, however, the present invention is not limited thereto but may employ other types of measuring instruments.

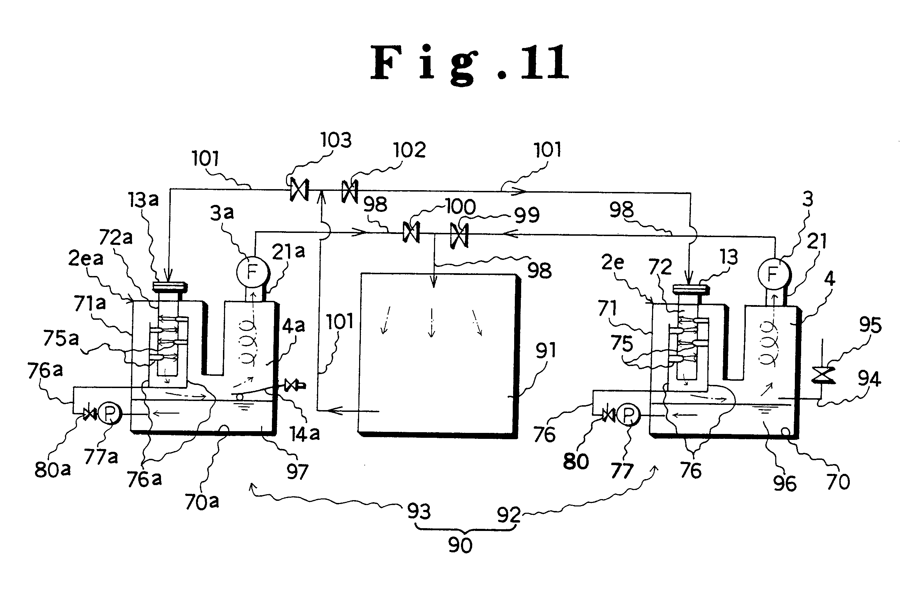

Fig. 11 illustrates an apparatus for embodying a method of utilizing liquid particles and negative ions according to the present invention. Specifically, a space disinfection and recovery apparatus 90 is provided for largely reducing a time necessary to disinfect an operation room 91 or the like (a space to be disinfected) from more than six hours required by a conventional like apparatus to within one hour. The apparatus 90 comprises a disinfecting unit 92 and a recovery unit 93 for recovering disinfecting liquid floating in the operation room 91. Since the disinfecting unit 92 is substantially identical in structure to the liquid particle and negative ions producing apparatus le shown in Fig. 5, the same elements in Fig. 11 as those of Fig. 5 will be designated by the same reference numerals. Also, since the disinfecting unit 92 is constructed in a similar manner to the recovery apparatus 93, the same elements will be designated by the same reference numerals, however, the reference numerals designating the elements of the recovery apparatus 93 are suffixed by a for distinction.

The disinfecting unit 92 differs from the apparatus le in Fig. 5 in that the former is provided with a disinfecting liquid supply path 94 equipped with a valve 95, in place of the ball tap valve 14 of the latter, so as to supply a tank 70 with a disinfecting liquid 96. On the other hand, the recovery apparatus 93 supplies a tank 70a with a potable water 97 or the like through a ball tap valve 14a, similarly to the apparatus 1e.

The foregoing operation room 91, the disinfecting unit 92 and the recovery unit 93 are connected in the following manner. Fans 3 and 3a of the respective disinfecting unit 92 and recovery unit 93 are connected to the operation room 91 through a pipe 98. This pipe is equipped with valves 99 and 100. The operation room 91 is connected to an air inlet port 13 of an air channel 72 of an fine liquid particle producing unit 2e in the disinfecting unit 92 and an air inlet port 13a of an air channel 72a of a fine water particle producing unit 2ea in the recovery unit 93 through a pipe 103. This pipe 103 is equipped with valves 102 and 103.

Next, a space disinfection and recovery method according to the present invention will be explained in connection with the space disinfection and recovery apparatus 90.

First, the valve 99 arranged in the pipe 98 is opened, the valve 100 arranged in the pipe 98 is closed, the valve 102 arranged in the pipe 101 is opened, and the valve 103 arranged in the pipe 101 is closed. Next, the valve 95 is opened to supply a disinfecting liquid 96 to the tank 70 of the fine liquid particle producing unit 2e in the disinfecting unit 92 up to a predetermined level from the disinfecting liquid supply path 94. After supplying the tank 70 with the disinfecting liquid 96, the valve 95 is closed, and the wind fan 3 and a pump 77 are rotated. The disinfecting liquid 96 is spouted through the supply path 76 from nozzles 75 to the air channel 72 and sputtered as fine disinfecting liquid particles, and simultaneously dispersed in the air introduced in the air channel 72 by the wind fan 3, thus generating fine disinfectant liquid particle mixed air containing positive ions and negative ions. Then, a separator 4 is driven to select air mixed with fine disinfecting liquid particles having a diameter of 2 $\mu$m or less in which negatively ionized air particles prevail. This mixed air containing fine disinfecting liquid particles enters from the air outlet port 21 to the operation room 91 through the pipe 98 and the valve 99. Then, the fine disinfecting liquid particle mixed air, after disinfecting the operation room 91, passes through the pipe 101 and the valve 102 and is again supplied through the air inlet port 13 to the air channel of the fine liquid particle producing unit 2e, thus performing a circulating operation for a predetermined time period to disinfect the operation room 91.

Since the floating disinfecting liquid particles must be recovered after the operation room 91 has been disinfected, the valve 99 is closed, the valve 100 is opened, the valve 102 is closed, and the valve 103 is opened. Next, the tank 70a of the recovery unit 93 is supplied with the potable water 97 through the ball tap valve 14, and the fan 3a and the pump 77a are rotated to generate ultra-fine water particle mixed air which contains negatively ionized ultra-fine water particles having the diameter of 1.0 $\mu$m or less in a manner similar to the operation of the disinfecting unit 92. This mixed air is supplied to the operation room 91 from the air outlet port 21a through the wind fan 3a, the pipe 98 and the valve 100. Then, the ultra-fine water particles capture and recover the disinfecting liquid particles floating in the operation room 91 and thereafter are again supplied to the air channel 72a of the fine water particle producing unit 2ea from the air inlet port 13a through the pipe 101 and the valve 103, thus performing a circulating operation for about thirty minutes to completely capture and recover the disinfecting liquid particles.

Incidentally, the above embodiment has been explained for the case where the operation room 91 is disinfected by the disinfecting unit of the present invention, and then the recovery unit 93 is used to recover particles of the disinfecting liquid 96 floating in the operation room 91. Alternatively, any appropriate means may be used to disinfect the operation room 91, and then particles of the disinfecting liquid 96 floating in the operation room 91 may be recovered by the recovery unit 93 of the present invention.

As described above in detail, the method of producing liquid particles and negative ions according to the present invention provides fine liquid particles having a diameter of 0.3 $\mu$m or less which can reach pulmonary alveoli by producing fine liquid particles by the fine liquid particle producing unit and mixing the fine particles with air supplied at a velocity of 0.5 - 50 m/sec to produce fine liquid particle mixed air which is then passed through the separator to remove fine liquid particles, the diameter of which is larger than 0.3 $\mu$m, while maintaining the above velocity, thereby producing ultra-fine liquid particle mixed air capable of reaching pulmonary alveoli. The ultra-fine liquid particles with a diameter of 0.3 $\mu$m or less, which can reach pulmonary alveoli, are expected to provide the humidification effects for the respiratory organs of human and therefore help a patient to easily spit out phlegm.

Also, the apparatus for utilizing liquid particles and negative ions may be used to dissolve medicaments, for example, antasthmatic, antibiotics and so on in a liquid and produce liquid particles capable of reaching pulmonary alveoli from this solution, thereby making it possible to directly supply the pulmonary alveoli of a patient with these medicaments such that the patient absorbs them from the pulmonary alveoli by breathing.

Further, when negative ions are principally utilized, a separator for separating fine liquid particles having the diameter of more than 1.0 $\mu$m may be selected to produce negative ions which are believed to produce a variety of effects as described above when the human is exposed thereto.

Furthermore, the ultra-fine liquid particle mixed air prevalently negatively ionized may be utilized to clean air in a room or the like with its fine particle capturing function.

It is also possible to prevent troubles caused by liquid particles and negative ions which can be unintentionally produced with a liquid other than water. Further, the ultra-fine particles may be used for disinfecting an operation room or the like and quickly recovered after the disinfection.

## Claims

1. A method of generating liquid particles and negative ions comprising the steps of:
   generating liquid particles from a liquid;
   mixing said liquid particles with flowing air to produce a mixture of air and liquid particles; and
   separating from said mixture liquid particles having a diameter greater than a predetermined value.

2. A method of generating liquid particles and negative ions comprising the steps of:
   generating fine liquid particles from a liquid by a fine liquid particle producing unit;
   simultaneously with said fine liquid particle generating step mixing said fine liquid particles with air at a velocity of 0.5 - 50 m/sec to produce fine liquid particle mixed air; and
   passing said fine liquid particle mixed air through a separator (4) substantially to remove fine particles, the diameter of which is larger than 0.3 $\mu$m to produce ultra-fine liquid particle mixed air.

3. A method as claimed in claim 2 wherein at least $5.0 \times 10^8$ or more of ultra-fine liquid particles with the diameter of 0.3 $\mu$m or less are produced per $1m^3$ of said ultra fine liquid particle air.

4. A method of producing liquid particles and negative ions, comprising the steps of:
   generating fine liquid particles from a liquid by a fine liquid particle producing unit;
   simultaneously with said fine liquid particle generating step mixing said fine liquid particles with air at a velocity of 0.5 - 50 m/sec to produce fine liquid particle mixed air;
   passing said fine liquid particle mixed air through a separator (4) substantially to remove fine particles, the diameter of which is larger than 1.0 $\mu$m.

5. A method as claimed in claim 4 wherein $1.25 \times 10^9$ or more of negative ions are produced per 1 $m^3$ of said ultra-fine liquid particle mixed air.

6. A method of utilizing liquid particles and negative ions comprising the steps of:
   supplying a room or space with the ultra-fine liquid particle mixed air containing ultra-fine liquid particles and negative ions having a diameter of 1.0 µm or less by a method as set forth in any of claims 1 to 5;

mixing said ultra-fine liquid particle mixed air with air in the room to be cleaned; and

circulatingly blowing said mixed air into fine liquid particles produced by a fine liquid particle producing unit at a velocity ranging from 5 to 50 m/sec to clean the air in the room or space.

7. A method of utilizing liquid particles and negative ions comprising the steps of:

preparing fine liquid particle mixed air with a disinfecting liquid;

passing said fine liquid particle mixed air through a separator (4) to substantially separate fine disinfecting liquid particles having diameters larger than a predetermined value, thereby producing ultra-fine disinfecting liquid particle mixed air;

filling a space (25) to be disinfected with said ultra-fine disinfecting liquid particle mixed air and circulating the same;

producing fine water particles from aseptic water in place of the disinfecting liquid after a lapse of a predetermined time period;

simultaneously blowing air into said fine water particles at a velocity ranging from 0.5 to 50 m/sec to prepare fine water particle mixed air;

passing said fine water particle mixed air through a separator substantially to separate fine water particles with a diameter of more than 2.0 µm to prepare ultra-fine water mixed air;

supplying said ultra-fine water particle mixed air into said space to be disinfected;

capturing disinfecting liquid particles remaining in said space with said ultra-fine water particles, thus recovering said disinfecting liquid particles.

8. Apparatus for generating liquid particles and negative ions comprising:

means (2) for generating liquid particles from a liquid;

means (3) for introducing to said particles moving air to produce a mixture of air and liquid particles; and

means (4) for separating from said mixture liquid particles having a diameter greater than a predetermined value.

9. An apparatus for producing liquid particles and negative ions comprising:

a fine liquid particle producing unit (2) for generating fine liquid particles from a liquid;

a fan (3) for introducing air at a velocity of 0.5 - 50 m/sec into said fine liquid particle producing unit (2) simultaneously with the generation of fine liquid particles by said fine liquid particle producing unit to produce fine liquid particle mixed air; and

a separator (4) for substantially separating fine liquid particles in said fine liquid particle mixed air, the diameter of which is larger than 0.3 µm, to produce ultra-fine liquid particle mixed air.

10. An apparatus for producing liquid particles and negative ions comprising:

a fine liquid particle producing unit (2) for generating fine liquid particles from a liquid;

a fan (3) for introducing air at a wind velocity of 0.5 - 50 m/sec into said fine liquid particle producing unit (2) simultaneously with the generation of fine liquid particles by said fine liquid particle producing unit to produce fine liquid particle mixed air; and

a separator (4) for substantially separating fine liquid particles in said fine liquid particle mixed air, the diameter of which is larger than 1.0 µm, to produce ultra-fine liquid particle mixed air.

11. An apparatus for producing liquid particles and negative ions according to claim 9 or 10, wherein said fine liquid particle producing unit comprises:

fine liquid particle generating means (44) including:

a tank (40) having an air inlet port (13) and an air outlet port (12);

at least one disk (41) arranged rotatably in the horizontal direction in said tank;

a liquid supply path (42) positioned above said disk and connected to said tank;

driving means (45) for rotating said disk; and

liquid supply means for supplying a liquid at a gage pressure ranging from -0.20 to 3.5 kg/cm$^2$ to said liquid supply path of said fine liquid particle generating means.

12. An apparatus for producing liquid particles and negative ions according to claim 9 or 10, wherein said fine liquid particle producing unit comprises:

a tank (10) having an air inlet port (B), an air outlet port (12) and a liquid supply path (14); and

an ultra-sonic humidifier (11) for producing fine liquid particles from a liquid contained in said tank.

13. An apparatus for producing liquid particles and negative ions according to claim 9 or 10, wherein said fine liquid particle producing unit comprises:

fine particle generating means (52) having an impeller (50) for rotating at a high speed to supply air and a liquid supply path (51); and

liquid supply means for supplying a liquid at a gage pressure ranging from -0.20 to 3.5 kg/cm$^2$ to said impeller of said fine liquid particle generating means through said liquid supply path.

14. An apparatus for producing liquid particles and negative ions according to claim 9 or 10, wherein said fine liquid particle producing unit comprises:

liquid spouting means (63) for spouting a liquid from a plurality of nozzles (62) having a diameter of 0.2 - 8 mm arranged around a spouting tube (61) mounted in a central portion inside said liquid spouting means to a lateral portion of said liquid spouting means spaced by 10 - 150 cm from said nozzles to generate an extremely large number of fine liquid particles; and

liquid supply means (64) for supplying a liquid at a gage pressure ranging from 0.5 to 3.5 kg/cm$^2$ from said nozzles.

15. An apparatus for producing liquid particles and negative ions according to claim 9 or 10, wherein said fine liquid particle producing unit comprises:

an air channel (72);

one or more nozzles (75) for spouting a liquid from one wall surface (73) onto the opposite wall surface (74) of said air channel; and

liquid supply means for supplying a liquid at a gage pressure ranging from 0.5 to 3.5 kg/cm$^2$ from said nozzles.

16. An apparatus for producing liquid particles and negative ions according to any of claims 9 to 15, wherein said fine liquid particle producing unit comprises dissolving means (30) for mixing a medicament with a liquid to provide a solution, wherein said fine liquid particle producing unit is provided with said solution to produce ultra-fine liquid particles containing said medicament.

17. An apparatus for producing liquid particles and negative ions according to any of claims 9 to 16 further comprising a water detecting sensor (113;115;116), wherein at least one of said fine liquid particle producing unit (2) and said fan (3) is rendered inoperative when said water detecting sensor determines that a liquid is not water.

# Fig.1

# Fig. 2

# Fig.3

# Fig. 4

EP 0 590 752 A1

Fig.5

# Fig.6

# Fig.7

Fig. 8

# Fig. 9

EP 0 590 752 A1

# Fig.10

EP 0 590 752 A1

Fig.11

EP 0 590 752 A1

**European Patent Office**

## EUROPEAN SEARCH REPORT

Application Number

EP 93 30 1477

### DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | PATENT ABSTRACTS OF JAPAN vol. 016, no. 413 (C-0980)2 September 1992 & JP-A-04 141 179 (IZUMI KENKIYUUSHIYO) 14 May 1992 * abstract * * figures * | 1-17 | A61M15/02 |
| Y | FR-A-2 654 273 (SEKHAVAT) * claims; figures * | 1,2,4, 6-11 | |
| Y | FR-A-1 242 534 (MIKOULINE) * the whole document * | 1,2,4, 6-11 | |
| A | US-A-3 232 292 (SCHAEFER) * claim 1 * | 1,8,9 | |
| A | EP-A-0 280 754 (SCHMIDT) * claims 1,8 * | 6,7,14, 15 | |
| A | FR-A-2 429 045 (CRAIGHERO) * claim 1 * | 6,7 | TECHNICAL FIELDS SEARCHED (Int.Cl.5) A61M A61N B05B |
| A | US-A-4 369 776 (ROBERTS) * abstract * | 17 | |

The present search report has been drawn up for all claims

| Place of search | Date of completion of the search | Examiner |
|---|---|---|
| THE HAGUE | 15 December 1993 | Villeneuve, J-M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

25